# EUROPEAN PATENT APPLICATION

(11) **EP 1 029 531 A2**
(43) Date of publication of application: **23.08.2000**
(21) Application number: 00200482.8
(22) Date of filing: 14.02.2000
(51) Int. Cl.: A61K 7/48

(54) **Vegetal extract as cosmetic agent for skin**

(30) Priority: 17.02.1999 JP 3882799
(71) Applicant: Bristol-Myers Squibb Company, New York, N.Y. 10154 (US)
(72) Inventor: Iawi, Shigeru, Yokohama-Shi, Kanagwa (JP); Kaneko, Teruhisa, Tokyo 170-0003 (JP); Nakashima, Nahoko, Ichikawa-shi, Chiba 272-0137 (JP); Nagase, Masaaki, Hachioji-shi, Tokyo 192-0021 (JP)
(74) Representative: Mays, Julie

(57) **Abstract**

The present invention is to offer an external agent for skin having a big effect of prevention of senescence of skin and head skin caused, for example, by exposure to ultraviolet ray. An extract of *Filipendula ulmaria* is used as an effective component of an external agent for skin.

## Description

The present invention relates to an external agent for skin which prevents senescence of skin and head skin resulted, for example, by exposure to ultraviolet ray.

### PRIOR ART

It is believed that an excessive stimulation to skin from outside such as exposure to ultraviolet ray for long time induces serious sick and wound such as photodermatosis, photosenescence and photocarcinogenesis. Especially, photosenescence which is a chronic accumulated photogenic damage has been clarified to be induced by denaturation of tissues in a molecular level such as cross-linking of collagen which is elastic fiber in dermis, changes in glucosaminoglucan, inactivation of antioxidative enzyme activity, oxidation of membrane lipids and activation of melanin production.

On the other hand, it has been pointed out that the antioxidative function which is inherent to living body such as a scavenging activity on the active oxygen species such as superoxide dismutase lowers together with aging and, in the skin where senescence proceeds, it is no longer possible to achieve a sufficient protective function against oxidative stimulation. As a result thereof, oxidative damage is accumulated in the skin whereupon wrinkles, spots, freckles, etc. are generated.

Accordingly, it is believed to be effective for protecting the skin from such oxidative damages to compound active oxygen scavenger, radical scavenger, tyrosinase inhibitor, et. With an external agent for skin and there have been reports for the external agents for skin compounded with various antioxidative vitamins, components derived from plants, carotenoids and synthetic chemicals.

Examples of the plant extract reported to have antioxidative effects are extracts of green tea leaves and of ginkgo leaves. Extract of green tea leaves contains polyphenols such as catechins while that of ginkgo leaves contains flanovoids and they have been reported to exhibit an effect of eliminating the superoxides. However, a protective effect of those extracts to skin tissues is not sufficient, their components are relatively unstable and color of the extracts is very dark and, therefore, those extracts have rarely been used practically as cosmetic components, etc.

As such, the components which have been proposed up to now are not satisfactory in view of their effect and stability and there has been a demand for developing new components which are highly stable and have good preventive effect to senescence.

### PROBLEMS TO BE SOLVED BY THE INVENTION

An object of the present invention is to offer an external agent for skin containing highly safe and novel effective components which exhibits good effect for inhibiting senescent phenomena of oxidation, inflammation, melanin production, etc. of skin and head skin induced by aging and oxidative stress such as exposure to ultraviolet ray, etc.

### MEANS TO SOLVE THE PROBLEMS

In order to solve the above-mentioned problems, the present inventors have conducted an intensive study for the substances which exhibit an effective eliminating effect to active oxygen species, radical scavenging effect and tyrosinase inhibiting effect, found that an extract of *Filipendula ulmaria* shows very high function in terms of all of the above effects and accomplished an external agent for skin for effectively preventing the senescence by compounding said extract.

Thus, the present invention relates to an external agent for skin where *Filipendula ulmaria* is used as an effective component.

*Filipendula ulmaria* is a plant belonging to genus *Filipendula*, is a species near *Filipendula multifunga* and is sometimes called meadowsweet or queen of the meadow.

An extract of *Filipendula ulmaria* used in the present invention is a product which is obtained by extracting root, seeds, petals, leaves or stem per se or dried or pulverized ones thereof with an appropriate amount of water, organic solvent or a mixture thereof. After the extracting operation, the extracted solution is usually filtered. The resulting filtrate, a concentrate thereof or an extract prepared by further extraction of this filtrate is usually used as an effective component of the external agent for skin of the present invention. If necessary, a concentrate prepared by concentrating the above by means of fractionation such as column chromatography may be compounded therewith.

Non-limitative examples of the extracting solvent are water; lower alcohol such as methanol, ethanol, n-propanol, isopropanol, 1-butanol, 2-butanol and tert-butanol; polyhydric alcohol such as 1,3-butylene glycol, 1,4-butylene glycol, 2,3-butylene glycol and propylene glycol; and a mixture thereof.

There is no particular limitation for the compounding amount of the extract of *Filipendula ulmaria* compounded in the external agent for skin of the present invention and compounding of 0.0001-10% by weight or, preferably, 0.001-5% by weight in the external agent for skin gives a valid effect.

The external agent for skin of the present invention may be prepared in a dosage form such as milky lotion, cosmetic lotion, cream and pack by known methods and it may be further compounded with known cosmetic materials such as surface-active agents, ultraviolet absorbers or ultraviolet scattering agents, antioxidants and whitening agents.

The external agent for skin of the present invention has a high safety and also shows significant antioxidative effect, active oxygen species scavenging effect, radical scavenging effect and tyrosinase activity inhibiting effect whereby it has a good effect of preventing senescence, wrinkles, chapping and dandruff induced by aging or by an excessive irradiation of ultraviolet ray and also has a good whitening effect by preventing spots and freckles.

### EXAMPLES

The present invention will now be further illustrated by way of the following examples although the present invention is never limited thereto.

### Example 1

### Manufacturing Example 1.

After 1 kg of dried seeds of *Filipendula ulmaria* were finely ground, 5 kg of a 50% by weight aqueous 1,3-butylene glycol was added thereto and the mixture was heated to reflux with stirring to conduct an extraction. The extract was filtered and the filtrate was concentrated to give about 50 g of a seed extract which was solid.

### Manufacturing Example 2.

After 1 kg of dried pedals of *Filipendula ulmaria* were finely ground, 5 kg of ethanol was added thereto and the mixture was heated to reflux with stirring to conduct an extraction. The extract was filtered and the filtrate was concentrated to give about 100 g of a petal extract which was solid.

### Manufacturing Example 3.

After 1 kg of dried root, dried leaves and dried stem of *Filipendula ulmaria* were combined followed by finely grinding, 10 kg of 50% (by weight) ethanol was added thereto and the mixture was heated to reflux with stirring to conduct an extraction. The extract was filtered and the filtrate was concentrated to give about 100 g of an extract of root, leaves and stem which was solid.

### Example 2

### Test Example 1. Scavenging Effect of an Extract of Filipendula ulmaria on the Active Oxygen Species.

As a scavenging effect on the active oxygen species, a test was done by conducting a reaction of nitro blue tetrazolium (NBT) with superoxide (SO) produced by oxidization of xanthine (XA) using xanthine oxidase (XOD). Thus, to 3 mL of a sodium carbonate buffer (pH: 10.2) were added each 0.1 mL of 0.75 mM NBT, 3.0 mM XA, 3.0 mM XOD and 3.0 mM EDTA solutions and an appropriate amount of an extract mentioned in any of Manufacturing Examples 1-3 was added thereto. To the mixture was added an appropriate amount of XOD and the reaction was started. Absorbance at 560 nm after ten minutes was measured. The ratio of an increase in the absorbance of the control where no extract was added to a suppressed increase in the absorbance by elimination of SO by an extract was calculated as an amount of elimination of SO by the extract. As shown in Table 1, each of the extracts of Manufacturing Examples 1-3 had an activity of efficient elimination of SO and the effect was from two- to six- fold of the catechin sample.

**Table 1**

| Sample (1 mg/mL added to the test system) | Rate of Eliminating the Superoxide (%) |
|---|---|
| Seed extract¹ | 90 |
| Petal extract ² | 85 |
| Root-leaf-stem extract³ | 30 |
| Catechin ⁴ | 15 |

| | |
|---|---|
| ¹ A product manufactured in Manufacturing Example 1 | |
| ² A product manufactured in Manufacturing Example 2 | |
| ³ A product manufactured in Manufacturing Example 3 | |
| ⁴ Manufactured by Wako Pure Chemical Industries, Ltd. | |

### Test Example 2. Effect of an Extract of Filipendula ulmaria for Inhibiting the Cross-Linking of Collagen.

Effect of inhibiting the cross-linking reaction of collagen which is a kind of elastic tissue of dermis by irradiation of ultraviolet ray was tested. Thus, an appropriate amount of an extract obtained in each of Manufacturing Examples 1-3 was added to collagen dissolved in an amount of about 0.3% by weight and the mixture was irradiated with ultraviolet ray (4J) using a UV lamp. After centrifuging at 5,000 rpm for 30 minutes at 4°C, the cross-linked collagen was separated. Protein content was measured by a Lowry method which is a common method. Inhibiting rate of the extract for the cross-linking of collagen was calculated on the basis that, when the amount of cross-linked collagen obtained by the same manner except that no extract was added, it was defined as a control. The result was that, as shown in Table 2, the extracts of Manufacturing Examples 1-3 strongly inhibited the cross-linking of collagen caused by irradiation of ultraviolet ray and the effect was from five- to tenfold of α-tocopherol.

**Table 2**

| Sample (1 mg/mL added to the test system) | Rate of Eliminating the Superoxide (%) |
|---|---|
| Seed extract¹ | 50 |
| Petal extract² | 53 |
| Root-leaf-stem extract³ | 28 |
| α-tocopherol⁵ | 5 |

| | |
|---|---|
| ¹ A product manufactured in Manufacturing Example 1 | |
| ² A product manufactured in Manufacturing Example 2 | |
| ³ A product manufactured in Manufacturing Example 3 | |
| ⁵ Manufactured by Wako Pure Chemical Industries, Ltd. | |

### Test Example 3. Effect of an Extract of Filipendula ulmaria For Preventing the UV Damage in the Skin Model

Freeze-dried pigskin was used as a model for human skin and an effect of protecting its damage caused by irradiation of ultraviolet ray was tested. Thus, sterilized and freeze-dried pigskin (ALLOASK; manufactured by Taiho Pharmaceutical Cp., Ltd.) was treated with trypsin and then an appropriate amount of each of the extracts obtained in Manufacturing Examples 1-3 was applied to the resulting corneal layer. After being irradiated with ultraviolet ray (4J) using a UV lamp, softness and degree of oxidization of the corneal layer were measured. With regard to the softness of the corneal layer, it was calculated in terms of tensile strength (gmf) per elongating rate (%) using a tensile tester. With regard to degree of oxidation of corneal layer, lipid was extracted with a mixed solvent of acetone and ether and a measurement was conducted by a thiobarbituric acid method which is a common method. The result was that, as shown in Table 3, it was apparent that each of the extracts mentioned in Manufacturing Examples 1-3 significantly inhibited a decrease in softness and production of oxides which were oxidative damages in corneal layer of skin caused by irradiation of ultraviolet ray.

**Table 3**

| Sample | Before Irradiation of UV | | After Irradiation of UV | |
|---|---|---|---|---|
| | Oxide⁶ | Softness⁷ | Oxide | Softness |
| Nothing added | 3 | 280 | 153 | 385 |
| Seed extract¹ | 2 | 272 | 5 | 277 |
| Petal extract² | 4 | 265 | 7 | 274 |
| Root-leaf-stem extract³ | 2 | 277 | 8 | 280 |

| | | | | |
|---|---|---|---|---|
| ⁶ The amount of oxide (nmol/mL) expressed in terms of the amount of TBARS and the less the value, the less the amount of the oxide. | | | | |
| ⁷ Softness is expressed by the tensile strength per elongating ratio and the less the value, the more the softness. | | | | |
| ¹ a product manufactured in Manufacturing Example 1 | | | | |
| ² a product manufactured in Manufacturing Example 2 | | | | |
| ³ a product manufactured in Manufacturing Example 3 | | | | |

### Text Example 4. Effect of an Extract of Filipendula ulmaria for Whitening.

HM4KO which was a melanoma cell derived from human being was used and an inhibiting effect against melanine production by its incubation was tested. Thus, HM3KO which was incubated to an extent of around 5 X 10⁵ by conventional method was collected by centrifugation and made into pellets. Then an incubating dish of 10 cm diameter to which a medium (an Eagle's medium containing 10% of fetal bovine serum) was added was sown with the pellets followed by incubating at 37°C for 24 hours. After that, each of the extracts mentioned in Manufacturing Examples 1-3 was added thereto to make the final concentration 1-1,000 µg/mL and the incubation was continued for six days more. Cells were collected by centrifugation and the amount of melanin therein was measured by a spectrophotometer. The result was that, as shown in Table 4, each of the extracts mentioned in Manufacturing Examples 1-3 significantly inhibited the blackening of HM4KO which was the cell derived from human being and the effect was superior to kojic acid and arbutin.

**Table 4**

| Sample (50 µg/mL added to the test system) | Whitening Effect (the more +, the higher the effect) |
|---|---|
| Nothing added | - |
| Seed extract¹ | +++ |
| Petal extract ² | ++++ |
| Root-leaf-stem extract ³ | ++ |
| Kojic acid⁸ | + |
| Arbutin⁸ | + |

| | |
|---|---|
| ¹ A product manufactured in Manufacturing Example 1 | |
| ² A product manufactured in Manufacturing Example 2 | |
| ³ A product manufactured in Manufacturing Example 3 | |
| ⁸ Manufactured by Wako Pure Chemical Industries, Ltd. | |

It is apparent from the above results that, when the extract obtained from *Filipendula ulmaria* which is an effective component of the present invention is applied to skin, the oxidative damage of skin induced, for example, by irradiation of ultraviolet ray is inhibited achieving a very good preventive action to senescence.

Examples of compounding of external agent for skin for preventing the senescence according to the present invention will be given in the following Example.

### Example 3

### Formulation Example 1. Cosmetic Lotion.

| | Compounded Components | % |
|---|---|---|
| (a) | Polyoxyethylene (20E.O.) sorbitan monolaurate | 1.2 |
| | Ethanol | 8.0 |
| | Antiseptic agent | q.s. |
| | Perfume | q.s. |
| (b) | Seed extract* | 5.0 |
| | Glycerol | 5.0 |
| | 1,3-Butylene glycol | 6.5 |
| | Pure water | balance |

| | | |
|---|---|---|
| * manufactured in Manufacturing Example 1 | | |

### (Manufacturing Method)

Each of the components (a) and (b) were mixed and dissolved. The components (a) and (b) were mixed to give a homogeneous cosmetic lotion.

### Formulation Example 2. Milky Lotion

| | Compounded Components | % |
|---|---|---|
| (a) | Squalane | 8.0 |
| | Glyceryl monostearace | 1.0 |
| | Polyoxyethylene (10E.O.) sorbitan | 1.0 |
| | Polyoxyethylene (60E.O.) sorbitan tetraoleate | 0.5 |
| | Stearic acid | 0.5 |
| | Behenyl alcohol | 0.5 |
| (b) | Petal extract* | 5.0 |
| | Carboxyvinyl polymer | 0.1 |
| | Sodium hydroxyide | 0.05 |
| | Ethanol | 5.0 |
| | Pure water | balance |
| (c) | Perfume | q.s. |

| | | |
|---|---|---|
| *manufactured in Manufacturing Example 2 | | |

### (Manufacturing Method)

The component (a) was mixed by heating and kept at 70°C. The component (b) was mixed by heating and kept at 70°C. To the component (b) was added the component (a) and the mixture was emulsified homogeneously. After cooled, the component (c) was added thereto followed by mixing homogeneously to give a milky lotion.

### Formulation Example 3. Cream

| | Compounded Components | % |
|---|---|---|
| (a) | Squalane | 15.0 |
| | Stearic acid | 5.0 |
| | Cetyl isooctanoate | 5.0 |
| | Polyoxyethylene (40E.O.) monostearate | 2.0 |
| | Behenyl alcohol | 0.5 |
| (b) | Root-leaf-stem extract* | 3.0 |
| | 1,3-Butylene glycol | 5.0 |
| | Pure water | balance |
| (c) | Perfume | q.s. |

| | | |
|---|---|---|
| *manufactured in Manufacturing Example 3 | | |

### (Manufacturing Method)

The component (a) was mixed by heating and kept at 70°C. To the component (b) was mixed by heating and kept at 70°C. To the component (b) was added the component (a) followed by adding the component (c) to give a cream.

### Formulation Example 4. Hair Tonic

| Compound Components | % |
|---|---|
| Ethanol | 70.0 |
| Petal extract* | 2.0 |
| Extract of Japanese green gentian *(Swertia japonica)* | 3.0 |
| Isopropylmethylphenol | 0.2 |
| Tocopherol acetate | 0.8 |
| Pantothenyl alcohol | 0.8 |
| Dipotassium glycyrrhizate | 0.2 |
| Perfume | q.s. |

| | |
|---|---|
| * a product manufactured in Manufacturing Example 2 | |

### (Manufacturing Method)

All components were poured into ethanol successively followed by mixing.

All of the cosmetic lotion of Formulation Example 1, the milky lotion of Formulation Example 2, the cream of Formulation Example 3 and the hair tonic of Formulation Example 4 had good stability with a lapse of time and, when applied to skin, they were able to prevent the senescence induced by oxidative stimulation such as ultraviolet ray from outside, i.e., "wrinkles", "spots", "freckles", etc., and regenerated to the original state of skin having transparency and maintained it.

## Claims

1. External agent for skin which comprises an extract of Filipendula ulmaria as an effective component.

2. External agent according to Claim 1 wherein the material for the extract is one or more selected from root, seeds, petals, leaves and stem of Filipendula ulmaria.

3. External agent according to Claim 1 or 2 wherein the amount of the extract is 0.0001-10% by weight.

4. External agent according to Claims 1 to 3 wherein the extract is obtained by using an extraction treatment comprising an addition of 1 to 99% by weight of one or more selected from water, organic solvents and a combination thereof.

5. External agent according to any of Claims 1 to 4 wherein the external agent for skin is an agent for preventing senescence of skin.
